Europäisches Patentamt

European Patent Office

Office européen des brevets

(19)

(11) Veröffentlichungsnummer: **0 057 862**

**A1**

(12) # EUROPÄISCHE PATENTANMELDUNG

(21) Anmeldenummer: 82100581.6

(22) Anmeldetag: 28.01.82

(51) Int. Cl.³: **C 07 C 119/055**
C 07 C 119/048, C 08 G 18/79

(30) Priorität: 05.02.81 US 232056

(43) Veröffentlichungstag der Anmeldung:
18.08.82 Patentblatt 82/33

(84) Benannte Vertragsstaaten:
BE DE FR GB IT NL

(71) Anmelder: Mobay Chemical Corporation
Penn Lincoln Parkway West
Pittsburgh, Pennsylvania 15205(US)

(72) Erfinder: Dunlap, Kenneth L.
20 Rosary Road
New Martinsville, WV 26155(US)

(72) Erfinder: Allen, Gary F.
811 Clearview Terrace
New Martinsville, WV 26155(US)

(74) Vertreter: Weber, Gerhard, Dr. et al,
BAYER AG Zentralbereich Patente, Marken und
Lizenzen
D-5090 Leverkusen, Bayerwerk(DE)

(54) Bei niedriger Temperatur lagerbeständiges, teilweise carbodiimidisiertes Methylenbis (phenylisocyanat), ein Verfahren zu seiner Herstellung und seine Verwendung als Ausgangsstoff für die Diisocyanat-Polyadditionsreaktion.

(57) Die vorliegende Erfindung betrifft ein flüssiges, teilweise carbodiimidisiertes Methylenbis(phenylisocyanat), in dem das betreffende Methylenbis(phenylisocyanat) mindestens 15 Gewichtsprozent des 2,4'-Isomeren enthält, ein Verfahren zu dessen Herstellung durch partielle Carbodiimidisierung der Isocyanat-Gruppen eines Methylenbis(phenylisocyanats), das mindestens 15 Gew.% des 2,4'-Isomeren enthält, sowie seine Verwendung als Ausgangsstoff für die Diisocyanat-Polyadditionsreaktion.

EP 0 057 862 A1

Mobay Chemical Corporation

Pittsburgh, PA 15205/USA

0057862

- 1 -

Bei niedriger Temperatur lagerbeständiges, teilweise
carbodiimidisiertes Methylenbis(phenylisocyanat),
ein Verfahren zu seiner Herstellung und seine Verwendung
als Ausgangsstoff für die Diisocyanat-Polyadditionsreaktion.

Nach dem Stand der Technik ist eine Vielfalt von Verfahren zur Herstellung von flüssigem, teilweise carbodiimidisierten Methylenbis(phenylisocyanat) bekannt. Der Begriff "Methylenbis(phenylisocyanat)", wie er hierin verwendet und auch in der Fachwelt allgemein anerkannt ist, bezeichnet Methylenbis(phenylisocyanat), das weniger als 5 Gewichts-% Polyisocyanat mit einer Funktionalität größer als 2 enthält und 4,4'-Methylenbis(phenylisocyanat), 2,4'-Methylenbis(phenylisocyanat) und 2,2'Methylenbis(phenylisocyanat) sowie Mischungen aus diesen umfaßt. Wie in der Fachwelt bekannt ist, ist Methylenbis(phenylisocyanat) normalerweis bei Raumtemperatur fest und muß bei seiner Verwendung zur Herstellung von Polyurethanen, die zellig oder nicht-zellig aufgebaut sind, erst zur Überführung in den flüssigen Zustand erhitzt werden. Aus diesem Grunde sind in der Fachwelt vielfältige Versuche unternommen worden, ein modifiziertes Methylenbis(phenylisocyanat) herzustellen, das flüssig und lagerbeständig ist.

Ein nach dem Stand der Technik vorgeschlagener Weg in diese Richtung besteht darin, das im wesentlichen reine Methylenbis(phenylisocyanat) in Gegenwart eines Trialkylphosphats zu erhitzen und dadurch ein flüssiges, teilweise Mo 2191 carbodiimidisiertes Isocyanat-Produkt herzustellen (vgl. z.B. die US-PSen 3 384 653 und 3 449 256).

Weiterhin wurde vorgeschlagen, daß beim Erhitzen von Methylenbis(phenylisocyanat) für sich allein, in Abwesenheit jeglichen Katalysators, ein teilweise carbodiimidisiertes Isocyanat entstehen kann, daß beim Stehen kristallisiert (vgl. z.B. die US-PS 3 152 162). Es wird angenommen, daß dieses auf die unkontrollierte Reaktion zurückzuführen ist, die während des Schrittes des Erhitzens stattfindet.

Schließlich wurde vorgeschlagen, flüssige, teilweise carbodiimidisierte Isocyanate herzustellen unter Einsatz von i) Phospholinoxid-Katalysatoren in Verbindung mit Katalysatorgiften (vgl. z.B. die US-PSen 4 014 935 und 4 088 665); ii) relativ geringen Mengen des Phospholinoxid-Katalysators (vgl. z.B. die US-PS 4 154 752); und iii) Katalysatoren ausgewählt aus der aus solchen organischen Isocyanaten bestehenden Gruppe, die Biuret-, Harnstoff-, Amido-, Urethan-, Allophanat-, Isocyanurat-, Uretdion- und Uretonimin-Gruppen enthalten (vgl. z.B. die US-PS 3 640 966).

Wenngleich die gemäß den genannten Patenten hergestellten Produkte in einigen Fällen wirtschaftliche Bedeutung erlangt haben, haftet ihnen jedoch noch immer der Nachteil einer relativ schlechten Tieftemperaturbeständigkeit an. Im allgemeinen neigen diese Produkte dazu, bei Temperaturen um 0°C zu kristallisieren. Während sich hierdurch in wärmeren Klimazonen keinerlei Schwierigkeiten ergeben, kann diese Neigung zur Kristallisation in kälteren Gegenden, in denen das Produkt möglichweise eine gewisse Zeit in Behältern gelagert wird, zu einem sehr ernsten Problem werden.

Mo 2191

Nach dem Stand der Technik sind auch Diisocyanate bekannt, die beständig und bei -5°C 48 h flüssig sind. Diese Isocyanate werden hergestellt durch Umsetzung spezieller Propylenglycole mit einem Methylenbis(phenylisocyanat), das mindestens 15 Gewichts% des 2,4'-Isomeren enthält (vgl. die US-PS 4 118 411), oder durch Umsetzung spezieller Polyoxyethylenglycole mit einem Methylenbis-(phenylisocyanat), das mindestens 20 Gewichts-% des 2,4'-Isomeren enthält.

Gegenstand der vorliegenden Erfindung ist ein flüssiges, teilweise carbodiimidisiertes Methylenbis-(phenylisocyanat), dadurch gekennzeichnet, daß das Methylenbis(phenylisocyanat) mindestens 15 Gewichts-% des 2,4'-Isomeren enthält.

Gegenstand der Erfindung ist auch ein Verfahren zur Herstellung eines flüssigen, teilweise carbodiimidisierten Methylenbis(phenylisocyanats) durch partielle Carbodiimidisierung der Isocyanat-Gruppen eines Methylenbis(phenylisocyanats), dadurch gekennzeichnet, daß ein Methylenbis(phenylisocyanat) eingesetzt wird, das mindestens 15 Gewichts-% des 2,4'-Isomeren enthält.

Gegenstand der Erfindung ist schließlich auch die Verwendung des flüssigen, teilweise carbodiimidisierten Methylenbis-(phenylisocyanats) als Ausgangsstoff für die Diisocyanat-Polyadditionsreaktion.

Im allgemeinen muß das beim erfindungsgemäßen Verfahren einzusetzende Methylenbis-(phenylisocyanat) mindestens 15 Gewichts-% des 2,4'-Isomeren enthalten. Während es theoretisch

Mo 2191

keine obere Grenze für die Menge des 2,4'-Isomeren gibt, die in dem Isocyanat vorhanden sein könnte, ist es in der Praxis, aufgrund des Angebots auf dem heutigen Isocyanat-Markt, im allgemeinen nicht möglich, einen Gehalt des 2,4'-Isomeren zu erhalten, der über 70 % hinausgeht. So enthält nach einer praktischen Regel das beim erfindungsgemäßen Verfahren verwendete Methylen-bis(phenylisocyanat) von etwa 15 bis etwa 70 Gewichts-%, vorzugsweise von 20 bis 70 Gewichts-%, des 2,4'-Isomeren, wobei der Rest aus dem 4,4'-Isomeren und dem 2,2'-Isomeren und/oder verschiedenen MDI-Dimeren besteht (das 2,2'-Isomere und etwaige Dimere sind im allgemeinen nur in Spurenmengen, d.h. weniger als 1 Gewichts-% vorhanden). Besonders bevorzugt wird ein Gehalt des 2,4'-Isomeren oberhalb von etwa 40 Gewichts-%.

Der Begriff "teilweise carbodiimidisiertes Methylenbis-(phenylisocyanat)", wie er hierin verwendet wird, bezeichnet ein Methylenbis(phenylisocyanat) mit einem Grad der Carbodiimidisierung von 1 bis 90 %. Der "Grad der Carbodiimidisierung" wird definiert als derjenige Prozentsatz, der in dem Ausgangs-Isocyanat vorhandenen Isocyanat-Gruppen, die im Laufe der Carbodiimidisierungsreaktion unter Entwicklung von Kohlenstoffdioxid in Carbodiimid-Gruppen umgewandelt werden. Wie nach dem Stand der Technik bekannt ist, kann der Grad der Carbodiimidisierung während der Reaktion durch die Menge des von der Reaktionsmischung entwickelten Kohlenstoffdioxids bestimmt werden. Diese volumetrisch bestimmbare Kohlenstoffdioxid-Menge liefert somit zu jeder Zeit eine Information über den erreichten Grad der Carbodiimidisierung. In einer bevorzugten Ausführungsform beträgt der Grad der Carbodiimidisierung von 5 bis 30 %.

Mo 2191

In bezug auf die technische Durchführung des Verfahrens zur Herstellung der carbodiimidisierten Isocyanate der vorliegenden Erfindung bestehen keine besonderen Auflagen hinsichtlich der Wahl des Verfahrens. So kann beispielsweise jedes der im Vorstehenden genannten Verfahren verwendet werden. Der Schlüssel zu der vorliegenden Erfindung liegt im Einsatz eines Methylenbis(phenylisocyanats), das mindestens 15 Gewichts-% des 2,4'-Isomeren enthält. Infolgedessen eignet sich jedes der Verfahren, die in den US-PSen 3 384 653, 3 449 256, 3 152 162, 4 014 935, 4 088 665, 4 154 752 und 3 640 966 beschrieben wurden, auf die hier Bezug genommen wird. Gegenwärtig werden die in den US-PSen 3 640 966 und 4 154 752 beschriebenen Arbeitsweisen bevorzugt, wobei das Verfahren der letztgenannten US-PS besonders bevorzugt wird.

Die flüssigen, teilweise carbodiimidisierten Methylenbis(phenylisocyanate) der vorliegenden Erfindung oder ihre Lösungen in carbodiimid-freien Polyisocyanaten sind wertvolle Ausgangsstoffe für die Diisocyanat-Polyadditionsreaktion und können zur Herstellung von Kunststoffen mit von hart bis elastisch und zellig oder nicht-zellig variabler Beschaffenheit verwendet werden sowie für den Einsatz bei der Herstellung von Lacken, Überzügen, Beschichtungen, Folien und Formteilen. Wegen ihrer niedrigen Gefrierpunkte können die Materialien bei ziemlicher Kälte transportiert und gelagert werden.

Die vorliegende Erfindung wird durch die folgenden Beispiele weiter erläutert, in denen, soweit nicht anders vermerkt, die Angabe "Teile" jeweils Gewichtsteile bezeichnet.

Mo 2191

## Beispiele

### Beispiel 1 bis 8

In diesen Beispielen wurde das 2,4'/4,4'-Isomerenverhältnis wie in Tabelle I angegeben variiert. Ein aliquoter Anteil von 150 Teilen des jeweiligen Isocyanats wurde in einen mit Kühler, Stickstoff-Gaseinleitungsrohr und mechanischem Rührer versehenen 1-1-Dreihalskolben gegeben. Der Kolben wurde mit Stickstoff gründlich gespült, und ein langsamer Stickstoffstrom wurde aufrechterhalten. In jedem Fall wurde die Probe auf 200°C erhitzt, und 0,004 Teile einer 3-proz. Lösung von 1-Methyl-1-oxopholin (Isomerengemisch) in Toluol wurden hinzugefügt. Nach etwa 4 h wurde die Reaktionsmischung auf eine Temperatur von 25°C bis 40°C abgeschreckt. Das Material wurde 7 d bei 25°C stehen gelassen, wonach der in Tabelle I angegebene NCO-Gehalt erhalten wurde.

Zwei Arten von Beständigkeitsprüfungen wurden zur Bestimmung der Gefriereigenschaften jeder Probe durchgeführt. Bei der ersten Methode wurde jede Probe in ein Reagensglas gegeben, das unter Stickstoffatmosphäre abgeschmolzen und in ein Temperaturbad bei 21°C, Haake Modell KT 33, eingetaucht wurde. Die Temperatur des Bades wurde alle zwei Tage um 2°C bis 3°C gesenkt. Die Gefriertemperatur wurde als diejenige Temperatur registriert, bei der die Kristallisation begann. Die mit dem angegebenen Bad erreichbare tiefste Temperatur lag bei -17°C.

Bei der zweiten Methode wurde jede der Proben in eine Flasche von 59 ml (2 fl.oz.) gegeben, die mit Stickstoff gespült und dann verschlossen wurde. Proben wurden in

Mo 2191

Kühlschränke gestellt, in denen die Temperaturen konstant auf -4°C und auf -18°C gehalten wurden. Die Anzahl der Tage, während derer die Probe flüssig blieb, wurde registriert.

Die erhaltenen Ergebnisse sind in Tabelle I aufgeführt.

Mo 2191

## Tabelle I

| Beispiel | MDI-2,4'/4,4'-Verhältnis | NCO im Endprodukt % | Test 1 Gefrier-temp. °C | Test 2 Probe blieb flüssig | |
|---|---|---|---|---|---|
| | | | | d bei -4°C | d bei -18°C |
| 1 | 3 : 97 | 29,2 | 0 | 2 | keinen |
| 2 | 10 : 90 | 27,7 | -10 | >84 | keinen |
| 3 | 20 : 80 | 28,3 | -14 | 7 | keinen |
| 4 | 30 : 70 | 28,4 | -17 | 7 | 4 |
| 5 | 40 : 60 | 28,8 | | >84 | 7 |
| 6 | 50 : 50 | 27,7 | noch flüssig bei -17°C | >84 | >84 |
| 7 | 58,4: 41,6 | 28,1 | | >84 | >84 |
| 8 | 68,8: 31,2 | 27,5 | | >84 | >84 |

0057862

<u>Beispiel 9</u>

Etwa 1000 Teile des in Beispiel 7 verwendeten Isocyanats wurden in einen 1-1-Dreihalskolben gegeben und mit 9 Teilen t-Butanol bei 215°C gemischt. Nach etwa 2 h wurde die Reaktionsmischung abgekühlt. Das Material wurde 11 d bei Raumtemperatur stehen gelassen, wonach es noch flüssig war und einen NCO-Gehalt von 28,14 % aufwies. Nach einer Lagerdauer von 11 d bei -17°C war das Material immer noch flüssig.

Wiewohl die Erfindung im Vorhergehenden zum Zwecke der Erläuterung im einzelnen beschrieben wurde, ist darauf hinzuweisen, daß die betreffenden Einzelheiten nur dem genannten Zweck dienen und daß im Rahmen der Erfindung, wie er durch die Ansprüche abgegrenzt ist, durchaus durch Fachleute Verfahrensänderungen vorgenommen werden können.

Mo 2191

P a t e n t a n s p r ü c h e

1. Flüssiges, teilweise carbodiimidisiertes Methylenbis-
(phenylisocyanat), dadurch gekennzeichnet, daß das
Methylenbis(phenylisocyanat) mindestens 15 Gewichts-%
des 2,4'-Isomeren enthält.

2. Zusammensetzung nach Anspruch 1, dadurch gekennzeichnet,
daß das Methylenbis(phenylisocyanat) mindestens 20 Gewichts-% des 2,4'-Isomeren enthält.

3. Zusammensetzung nach Anspruch 1, dadurch gekennzeichnet,
daß das Methylenbis(phenylisocyanat) von etwa 15 bis
etwa 70 Gewichts-% des 2,4'-Isomeren enthält.

4. Zusammensetzung nach Anspruch 3, dadurch gekennzeichnet,
daß das Methylenbis(phenylisocyanat) von etwa 20 bis
etwa 70 Gewichts-% des 2,4'-Isomeren enthält.

5. Zusammensetzung nach Anspruch 4, dadurch gekennzeichnet,
daß das Methylenbis(phenylisocyanat) 40 % oder mehr
des 2,4'-Isomeren enthält.

6. Verfahren zur Herstellung eines flüssigen, teilweise
carbodiimidisierten Methylenbis(phenylisocyanats) durch
partielle Carbodiimidisierung der Isocyanat-Gruppen
eines Methylenbis(phenylisocyanats), dadurch gekennzeichnet, daß ein Methylenbis(phenylisocyanat) eingesetzt wird, das mindestens 15 Gewichts-% des 2,4'-Iso-
meren enthält.

Mo 2191

7. Verfahren nach Anspruch 6, dadurch gekennzeichnet, daß ein Methylenbis(phenylisocyanat) eingesetzt wird, das mindestens 20 Gewichts-% des 2,4'-Isomeren enthält.

8. Verfahren nach Anspruch 6, dadurch gekennzeichnet, daß ein Methylenbis(phenylisocyanat) eingesetzt wird, das von etwa 15 bis etwa 70 Gewichts-% des 2,4'-Isomeren enthält.

9. Verfahren nach Anspruch 8, dadurch gekennzeichnet, daß ein Methylenbis(phenylisocyanat) eingesetzt wird, das von etwa 20 bis etwa 70 Gewichts-% des 2,4'-Isomeren enthält.

10. Verwendung des flüssigen, teilweise carbodiimidisierten Methylenbis-(phenylisocyanats) gemäß Anspruch 1 bis 5 als Ausgangsstoff für die Diisocyanat-Polyadditions-reaktion.

Mo 2191

## Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A1 - 2 742 275 (ICI) <br> * Ansprüche 1, 13; Beispiele 3, 4, 6 * <br> -- | 1,6,10 |
| P,X | EP - A1 - 0 032 011 (ICI) <br> * Anspruch 3; Beispiele 13, 14 * <br> -- | 1,2,3, 5-8 |
| Y | GB - A - 1 510 424 (ICI) <br> * Ansprüche * <br> -- | 1 |
| Y | Chemical Abstracts Band 80, Nr. 25 <br> 24. Juni 1974 <br> Columbus, Ohio, USA <br> V.G. GOLOV et al. "Stability of methylene-bis(phenylene)diisocyanate during storage" <br> Seite 405, Spalte 1, Abstract Nr. 145684n <br> & Zh. Prikl. Khim. Band 47, Nr. 3, 1974 <br> Seiten 686 bis 687 <br> -- | 1 |
| A | EP - A1 - 0 005 233 (MOBAY) <br> * Patentansprüche * <br> -- | 1,6,10 |
| A | DE - A1 - 2 930 411 (BAYER) <br> * Beispiel 1 * <br> -- | 1 |
| A | DE - A1 - 2 810 596 (MOBAY) <br> ---- | |

### KLASSIFIKATION DER ANMELDUNG (Int Cl.³)

C 07 C 119/055
C 07 C 119/048
C 08 G   18/79

### RECHERCHIERTE SACHGEBIETE (Int. Cl.³)

C 07 C 119/042
C 07 C 119/048
C 07 C 119/055
C 08 G   18/76
C 08 G   18/79

### KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 27-04-1982 | PHILLIPS |

EPA form 1503.1   06.78